# EUROPEAN PATENT SPECIFICATION

(11) **EP 4 130 203 B1**
(45) Date of publication and mention of the grant of the patent: **04.09.2024**
(21) Application number: 22185032.4
(22) Date of filing: 14.07.2022
(51) Int. Cl.: C07C 7/04, C10G 70/02, C10G 45/44, C07C 5/10, C10G 9/36, C10G 65/16

(54) **PROCESS FOR SATURATING AROMATICS IN A PYROLYSIS STREAM**
VERFAHREN ZUR SÄTTIGUNG VON AROMATEN IN EINEM PYROLYSESTROM
PROCÉDÉ DE SATURATION D'AROMATIQUES DANS UN FLUX DE PYROLYSE

(30) Priority: 16.07.2021 US 202163222797 P
(43) Date of publication of application: 08.02.2023
(73) Proprietor: UOP LLC, Des Plaines, Illinois 60017-5017 (US)
(72) Inventor: MAHAPATRA, Nirlipt, Charlotte, 28202 (US); MATHUR, Ashish, Charlotte, 28202 (US); SARKAR, Prasenjit Basu, Charlotte, 28202 (US); RAJAPPAN, Rajesh, Charlotte, 28202 (US); FREY, Stanley Joseph, Charlotte, 28202 (US); HOEHN, Richard K., Charlotte, 28202 (US); JANI, Priyesh Jayendrakumar, Charlotte, 28202 (US); JAIN, Amit, Charlotte, 28202 (US)
(74) Representative: Boult Wade Tennant LLP

(56) References cited:
- WO-A1-2005/080530
- WO-A1-2021/009666
- US-A1- 2003 205 504
- US-A1- 2015 129 461
- US-A1- 2016 264 886
- US-A1- 2019 055 483

## Description

### FIELD

The field is processes for increasing the concentration of naphthenes in a feed stream.

### BACKGROUND

Ethylene and propylene are important chemicals for use in the production of other useful materials, such as polyethylene and polypropylene. Polyethylene and polypropylene are two of the most common plastics found in use today and have a wide variety of uses. Uses for ethylene and propylene include the production of vinyl chloride, ethylene oxide, ethylbenzene and alcohol.

The great bulk of the ethylene consumed in the production of the plastics and petrochemicals such as polyethylene is produced by the thermal cracking of hydrocarbons. Steam is usually mixed with the feed stream to the cracking furnace to reduce the hydrocarbon partial pressure and enhance olefin yield and to reduce the formation and deposition of carbonaceous material in the cracking reactors. The process is therefore often referred to a steam cracking or pyrolysis.

Steam cracking generates less valuable by-products such as pyrolysis gas (pygas) and fuel oil. Pygas contains large proportions of paraffins and aromatics. Paraffins can be recovered or further processed to prepare useful steam cracking feed. Aromatics are a very poor steam cracking feed because they typically increase the yield of low-value fuel oil.

An efficient process for managing aromatics in a pygas feed is needed for improving the value of steam cracking units.
US 2019/055483 A1 relates to a process for producing olefins using aromatic saturation. WO 2021/009666 A1 relates to a system and method for producing un-hydrogenated and hydrogenated C9+ compounds.
US 2016/264886 A1 relates to a process and apparatus for hydroprocessing and cracking hydrocarbons.
WO 2005/080530 A1 relates to an improved olefin plant recovery system employing a combination of catalytic distillation and fixed bed catalytic steps.
US 2003/205504 A1 relates to hydrodesulfurization of gasoline fractions.
US 2015/129461 A1 relates to apparatuses and methods for hydrotreating coker kerosene.

### BRIEF SUMMARY

The invention is in accordance with the appended claims. The disclosed process provides for saturation of a pyrolysis stream while managing the resulting exotherms. The process splits the pyrolysis stream into at least two feed streams for at least two saturation reactors. The process splits the hydrogen stream into at least two feed streams for the at least two saturation reactors. A recycle stream is also provided to manage the exotherm. The feed may comprise at least 5 wt% and preferably at least 10 wt% aromatics.

Additional details and embodiments of the disclosure will become apparent from the following detailed description of the disclosure.

### BRIEF DESCRIPTION OF THE DRAWINGS

FIG. 1 is a schematic view of a conversion unit of the present disclosure.
FIG. 2 is an alternative schematic view of the unit of FIG. 1.
FIG. 3 is an alternative schematic view of the unit of FIG. 2.

### DEFINITIONS

The term "communication" means that fluid flow is operatively permitted between enumerated components, which may be characterized as "fluid communication". The term "communication" may also mean that data or signals are transmitted between enumerated components which may be characterized as "informational communication".

The term "downstream communication" means that at least a portion of fluid flowing to the subject in downstream communication may operatively flow from the object with which it fluidly communicates.

The term "upstream communication" means that at least a portion of the fluid flowing from the subject in upstream communication may operatively flow to the object with which it fluidly communicates.

The term "direct communication" means that fluid flow from the upstream component enters the downstream component without passing through any other intervening vessel.

The term "indirect communication" means that fluid flow from the upstream component enters the downstream component after passing through an intervening vessel.

The term "bypass" means that the object is out of downstream communication with a bypassing subject at least to the extent of bypassing.

The term "column" means a distillation column or columns for separating one or more components of different volatilities. Unless otherwise indicated, each column includes a condenser on an overhead of the column to condense and reflux a portion of an overhead stream back to the top of the column and a reboiler at a bottom of the column to vaporize and send a portion of a bottoms stream back to the bottom of the column. Feeds to the columns may be preheated. The top pressure is the pressure of the overhead vapor at the vapor outlet of the column. The bottom temperature is the liquid bottom outlet temperature. Overhead lines and bottoms lines refer to the net lines from the column downstream of any reflux or reboil to the column. Stripper columns may omit a reboiler at a bottom of the column and instead provide heating requirements and separation impetus from a fluidized inert media such as steam. Stripping columns typically feed a top tray and take main product from the bottom.

As used herein, the term "a component-rich stream" means that the rich stream coming out of a vessel has a greater concentration of the component than the feed to the vessel.

As used herein, the term "a component-lean stream" means that the lean stream coming out of a vessel has a smaller concentration of the component than the feed to the vessel.

As used herein, the term "boiling point temperature" means atmospheric equivalent boiling point (AEBP) as calculated from the observed boiling temperature and the distillation pressure, as calculated using the equations furnished in ASTM D1160 appendix A7 entitled "Practice for Converting Observed Vapor Temperatures to Atmospheric Equivalent Temperatures".

As used herein, the term "True Boiling Point" (TBP) means a test method for determining the boiling point of a material which corresponds to ASTM D-2892 for the production of a liquefied gas, distillate fractions, and residuum of standardized quality on which analytical data can be obtained, and the determination of yields of the above fractions by both mass and volume from which a graph of temperature versus mass % distilled is produced using fifteen theoretical plates in a column with a 5:1 reflux ratio.

As used herein, the term "T5" or "T95" means the temperature at which 5 mass percent or 95 mass percent, as the case may be, respectively, of the sample boils using ASTM D-86 or TBP.

As used herein, the term "initial boiling point" (IBP) means the temperature at which the sample begins to boil using ASTM D-7169, ASTM D-86 or TBP, as the case may be.

As used herein, the term "end point" (EP) means the temperature at which the sample has all boiled off using ASTM D-7169, ASTM D-86 or TBP, as the case may be.

As used herein, the term "separator" means a vessel which has an inlet and at least an overhead vapor outlet and a bottoms liquid outlet and may also have an aqueous stream outlet from a boot. A flash drum is a type of separator which may be in downstream communication with a separator that may be operated at higher pressure.

As used herein, the term "predominant" or "predominate" means greater than 50%, suitably greater than 75% and preferably greater than 90%.

The term "Cx" is to be understood to refer to molecules having the number of carbon atoms represented by the subscript "x". Similarly, the term "Cx-" refers to molecules that contain less than or equal to x and preferably x and less carbon atoms. The term "Cx+" refers to molecules with more than or equal to x and preferably x and more carbon atoms.

### DETAILED DESCRIPTION

The present disclosure endeavors to convert aromatics rich streams to make them suitable for steam cracking feed. For example, aromatics in pygas can be saturated into naphthenes that may be recycled to the steam cracking unit and then cracked into useful olefins. Steam cracking disadvantageously converts aromatics to fuel oil, but naphthenes can be steam cracked to valuable light olefins. Saturation of aromatics can generate large amounts of heat which must be managed. The process may also be suitable for other pure aromatics or aromatics-rich streams in addition to pygas or pygases other than steam cracked pygas. The disclosed process increases the concentration of naphthenes and decreases the concentration of aromatics while the concentration of normal and iso-paraffins remain essentially unchanged.

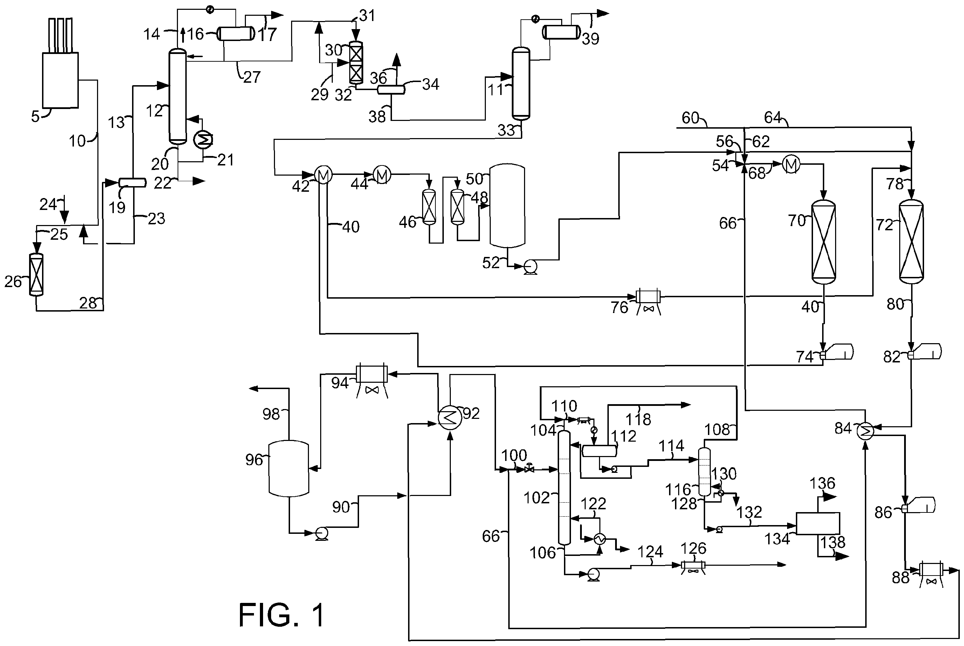

Turning to FIG. 1 of the present process, a pyrolysis stream in pyrolysis line 10 from a steam cracking unit 5 predominantly comprises C5+ hydrocarbons. The pyrolysis stream in line 10 may be subjected to selective hydrogenation before it is ready for saturation. The pyrolysis stream in line 10 may be subjected to selective hydrogenation to convert diolefins and conjugated-diolefins in the pyrolysis line 10 to monoolefins. A recycle stream in line 23 and hydrogen from a hydrogen line 24 is added to the pyrolysis stream in line 10 and a resulting combined pyrolysis stream in line 25 is charged to a selective hydrogenation reactor 26. The selective hydrogenation reactor 26 is normally operated at relatively mild hydrogenation conditions. The reactants will normally be maintained under the minimum pressure sufficient to maintain the reactants as liquid phase hydrocarbons. A broad range of suitable operating pressures therefore extends from 276 kPa(g) (40 psig) to 5516 kPa(g) (800 psig), or 345 kPa(g) (50 psig) to 3795 kPa(g) (550 psig). A relatively moderate temperature between 25°C (77°F) and 350°C (662°F), or 50°C (122°F) and 150°C (302°F) is typically employed. The liquid hourly space velocity of the reactants through the selective hydrogenation catalyst should be 1.0 hr⁻¹ and 35.0 hr⁻¹. To avoid the undesired saturation of a significant amount monoolefinic hydrocarbons, the mole ratio of hydrogen to diolefinic hydrocarbons in the material entering the bed of selective hydrogenation catalyst is maintained between 0.75:1 and 2.5:1.

Any suitable catalyst which is capable of selectively hydrogenating diolefins in a naphtha stream may be used. Suitable catalysts include, but are not limited to, a catalyst comprising platinum, palladium, copper, titanium, vanadium, chrome, manganese, cobalt, nickel, zinc, molybdenum, and cadmium or mixtures thereof. The metals are preferably supported on inorganic oxide supports such as silica and alumina, for example.

The selectively hydrogenated pyrolysis stream in line 28 is separated in a separator 19 to provide the recycle stream in a bottoms line 23 and a vaporous pyrolysis stream in an overhead line 13 which is fed to a pyrolysis fractionation column 12.

The vaporous pyrolysis stream in line 13 may be preliminarily fractionated in a pyrolysis fractionation column 12 to remove C9+ hydrocarbons. Light olefins preferably are removed from the pyrolysis stream in line 10 in a steam cracking light olefin recovery section prior to selective hydrogenation and pyrolysis fractionation. The pyrolysis fractionation column 12 is operated to separate an off gas stream in line 17 comprising C4- hydrocarbons wet gas, a net overhead stream comprising C5-C8 hydrocarbons pyrolysis product in a net overhead line 27, rich in benzenes, toluene and xylenes, and a pyrolysis bottoms stream rich in C9+ hydrocarbons in line 20. The pyrolysis overhead stream is withdrawn from the pyrolysis fractionation column 12 in an overhead line 14, condensed in a cooler and fed to a separator 16. A portion of the condensed pyrolysis overhead stream is recycled to the pyrolysis fractionation column 12 as reflux through a reflux line and the remaining portion of the condensed net pyrolysis overhead stream is withdrawn through a net pyrolysis overhead line 27. Wet gases are withdrawn in the off gas line 17 while the C5-C8 hydrocarbon pyrolysis product is withdrawn in the net overhead line 27.

The pyrolysis bottoms stream is withdrawn from pyrolysis fractionation column 12 through a bottoms line 20 where a portion of the pyrolysis bottoms stream flows through a reboiler line 21 to a reboiler heater and returns heated to the pyrolysis fractionation column 12. A net pyrolysis bottoms stream flows through line 22 rich in C9+ hydrocarbons which may be recovered or further processed. The pyrolysis fractionation column 12 operates in bottoms temperature range of 225 to 275°C and an overhead pressure of 250 to 350 kPa (gauge).

The combined pyrolysis product stream in line 31 may be heated and charged to the hydrotreating reactor 30. The hydrotreating reactor 30 may have one or more beds of hydrotreating catalyst to hydrodemetallate, hydrodenitrogenate and hydrodesulfurize the combined selectively hydrogenated pyrolysis stream. The combined pyrolysis product stream may be charged to the hydrotreating reactor 30 at a hydrotreating inlet temperature that may range from 200°C (392°F) to 400°C (752°F). The hydrotreating reactor 30 may employ interbed hydrogen quench streams from the hydrogen manifold 29.

Suitable hydrotreating catalysts are any known conventional hydrotreating catalysts and include those which are comprised of at least one Group VIII metal, preferably iron, cobalt and nickel, more preferably cobalt and/or nickel and at least one Group VI metal, preferably molybdenum and tungsten, on a high surface area support material, preferably alumina. Other suitable hydrotreating catalysts include zeolitic catalysts, as well as noble metal catalysts where the noble metal is selected from palladium and platinum. It is within the scope of the present description that more than one type of hydrotreating catalyst be used in the same hydrotreating reactor 30. The Group VIII metal is typically present in an amount ranging from 2 to 20 wt%, preferably from 4 to 12 wt%. The Group VI metal will typically be present in an amount ranging from 1 to 25 wt%, preferably from 2 to 25 wt%. Generally, hydrotreating conditions include a pressure of 700 kPa (100 psig) to 21 MPa (3000 psig). The hydrotreating outlet temperature may range between 300°C (572°F) and 427°C (800°F).

The hydrotreated effluent stream may exit the hydrotreating reactor in line 32 and enter a hydrotreating separator 34 to provide an overhead stream rich in hydrogen in line 36 that may be scrubbed (not shown) to remove hydrogen sulfide and ammonia or other compounds and compressed and returned back to the hydrogen manifold 29 and the hydrogen line 24 after perhaps supplementation with a make-up hydrogen stream. A hydrotreated pyrolysis stream is provided from a bottoms line 38 from the hydrotreater separator 34 and stripped in a stripper column 11 to remove C4- off gases in a stripper off-gas line 39 and a stripped pyrolysis stream in a stripper bottoms line 33.

The stripped pyrolysis stream may in line 33 is rich in C6-C8 aromatics. In an embodiment, the preliminary overhead feed comprises at least 5 to 10 wt% C6-C8 aromatics. The stripped pyrolysis stream may have the composition shown in Table 1.

**Table 1**

| COMPONENT | wt% |
|---|---|
| Isopentane | 3-7 |
| N-Pentane | 5-9 |
| Cyclopentane | 5-10 |
| N-Hexane | 1-5 |
| Cyclohexane | 4-9 |
| Benzene | 20-40 |
| N-Heptane | 0.1-1 |
| Methylcyclohexane | 0.5-3 |
| Toluene | 10-30 |
| 1,1-Dimethylcyclohexane | 0.5-3 |
| Ethylbenzene | 2-12 |
| Xylenes | 2-10 |
| C9+ | 1-5 |
| Total Aromatics | 34-92 |

The pyrolysis stream may comprise 12 to 26 wt% pentanes, 5 to 14 wt% hexane, 20 to 40 wt% benzene, 0.6 to 4 wt% heptanes, 10 to 30 wt% toluene, 2 to 12 wt% ethylbenzene, 2 to 10 wt% xylenes and 34 to 92 wt% total aromatics.

The hydrotreated pyrolysis stream in line 38 may have a sulfur concentration of below 0.5 wppm, but the saturation catalyst may be very sensitive to sulfur. Hence the hydrotreated pyrolysis stream is further desulfurized by passing it through sulfur guard beds 46 and 48 set up in a lead-lag arrangement and operated at hot conditions of 149°C (300°F) to 204°C (400°F). Low temperature sulfur guard beds may be used as well depending on the species of sulfur. The hydrotreated pyrolysis stream in line 33 is heated by heat exchange with a first saturated effluent stream in line 40 in a fresh feed reactor effluent exchanger 42 and then may be further heated in a guard bed charge heater 44 before it is fed to the sulfur guard beds 46 and 48 to have sulfur removed down to less than 0.1 wppm and preferably, 0.025 to 0.05 wppm sulfur. The hydrotreated pyrolysis stream in line 33 may also be heated by heat exchange with a second saturated effluent stream in line 80 in the fresh feed reactor effluent exchanger 42, but this embodiment is not shown.

The hot desulfurized pyrolysis stream from the guard beds 46 and 48 is routed to a feed surge drum 50, which may be blanketed by makeup gas and/or separator off gas. Further, this hot desulfurized pyrolysis stream in line 52 is pumped to the saturation reactor pressure of 1.7 MPa (250 psig) to 4.5 MPa (650 psig) by a charge pump.

The saturation of aromatics is extremely exothermic. Managing the exothermicity is conducted by splitting the pyrolysis stream in line 52 into multiple streams each for a dedicated saturation reactor. This arrangement can maintain the exotherm for each saturation reactor below 200°C (360°F), preferably below 111°C (200°F). The desulfurized pyrolysis stream in line 52 is split into at least a first pyrolysis stream in line 54 and a second pyrolysis stream in line 56.

Hydrogen partial pressure should be minimized so as to avoid side reactions such as hydrocracking reactions. To reduce the tendency for side reactions, a hydrogen stream in line 60 is split into a number of streams, perhaps like the number of multiple pyrolysis streams, each dedicated to a single saturation reactor. A hydrogen stream in line 60 entering the process is split into a first hydrogen stream in line 62 and a second hydrogen stream in line 64. The first hydrogen stream in line 62 is added to the first pyrolysis stream in line 54. The second hydrogen stream in line 64 is added to the second pyrolysis stream in line 56. For systems using a platinum-based catalyst, the hydrogen should be very pure make-up hydrogen, preferably from a pressure-swing adsorption unit, with no more than 1 mole-ppm carbon monoxide and no more than 0.5 mole-ppm hydrogen sulfide, preferably no more than 0.1 mole-ppm hydrogen sulfide. The saturation process can endure a higher concentration of impurities in the hydrogen if the operating temperature and/or the outlet hydrogen-to-hydrocarbon ratio is increased. Hydrogen with some concentration of light paraffinic gases can also be used provided the applicable contaminant limit is met for the catalyst.

The first pyrolysis stream in line 54 may comprise 5 to 50 vol-% and typically 30 to 50 vol-% of the hot desulfurized pyrolysis stream in line 52. The second pyrolysis stream in line 56 may comprise the balance. A third pyrolysis stream to a third saturation reactor is also contemplated but not shown. A third hydrogen stream to a third saturation reactor is also contemplated but not shown. The first hydrogen stream in line 62 is added to the first pyrolysis stream in line 54. Moreover, to further manage the saturation exotherm a recycle stream in line 66 is added to the first pyrolysis stream in line 54 to provide a first combined charge stream in line 68. The recycle stream is taken from a second saturated effluent stream in line 80. The first combined charge stream in line 68 may be heated to 120°C (248°F) to 230°C (446°F) and charged to the first saturation reactor 70.

In the first saturation reactor 70, aromatics are saturated over a bed of saturation catalyst to naphthenes to provide a first saturated effluent stream. The saturation catalyst may be the same in both saturation reactors 70 and 72. The saturation catalyst may comprise a noble metal, platinum or palladium, a platinum-lithium or nickel on a porous carrier material or any know commercial hydrogenation catalyst.

The porous carrier material may have a surface area of 25 to 500 square meters per gram, preferably 150 to 225 square meters per gram, and may comprise non-acidic, amorphous alumina. Gamma alumina may be preferred. In addition, a preferred alumina will have an apparent bulk density of 0.30 to 0.70 gm/cc and surface area characteristics such that the average pore diameter is 20 to 300 Angstroms and the pore volume is 0.10 to 1.0 milliliter per gram. The alumina carrier may be prepared by adding a suitable alkaline reagent, such as ammonium hydroxide, to a salt of aluminum, such as aluminum chloride, or aluminum nitrate, in an amount to form an aluminum hydroxide gel which, upon drying and calcination, is converted to alumina. The carrier material may be formed in any desired shape such as spheres, pills, cakes, extrudates, powders, granules, etc., and may further be utilized in any desired size.

The Group VIII noble metal component, for example platinum, may exist within the final catalytic composite as a compound such as an oxide, sulfide, halide, or in an elemental state. The Group VIII noble metal component generally comprises 0.01% to 2.0% by weight of the final composite, calculated on an elemental basis. The Group VIII noble metal component may be incorporated within the catalytic composite in any suitable manner including co-precipitation or cogellation with the carrier material, ion-exchange, or impregnation. Following impregnation, the composite may generally be dried at a temperature of 93°C (200°F) to 204°C (400°F), for a period of from 2 to 24 hours, or more, and finally calcined at a temperature of 371°C (700°F) to 538°C (1000°F), in an atmosphere of air, for a period of 0.5 to 10 hours.

In order to avoid side reactions which results in the loss of naphthenes, an alkalinous metal component may be combined with the catalytic composite in an amount of from 0.01% to 1.5% by weight. This component is selected from the group of alkali metals, particularly lithium and/or potassium.

The saturation catalyst may be reduced in a water-free environment after calcination to reduce the noble metal component. Moreover, the saturation catalyst may be presulfided such as in the presence of hydrogen sulfide to activate the catalyst.

A first saturated effluent stream exits the first saturation reactor 70 in the first saturated effluent line 40 with a greater concentration of naphthenes and a lower concentration of aromatics than in the first combined charge stream in line 68. The first saturated effluent stream in line 40 may be cooled in a first steam generator 74, then by heat exchange with the hydrotreated pyrolysis stream in the hydrotreater separator bottoms line 38 in the fresh feed reactor effluent exchanger 42 and then in a first reactor effluent cooler 76.

The second hydrogen stream in line 64 is added to the second pyrolysis stream in line 56. Moreover, to manage the exotherm in the second saturation reactor 72, the cooled first saturated effluent stream in line 40 is also added to the second pyrolysis stream in line 56 to provide a second combined charge stream in line 78. The second combined charge stream in line 78 may be at a temperature of 120°C (248°F) to 230°C (446°F) and charged to the second saturation reactor 72.

In the second saturation reactor 72 aromatics are saturated over a bed of saturation catalyst to naphthenes to provide a second saturated effluent stream. The saturation catalyst may be the same in both saturation reactors 70 and 72. The saturation catalyst may comprise a porous carrier material having combined therewith a Group VIII noble metal component or any commercial hydrogenation catalyst as described for the first saturation reactor 70.

Conditions in the saturation reactors 70 and 72 should include a hydrogen to hydrocarbon mole ratio of 0.01 to 2, preferably 0.025 to 0.5 at the reactor outlet, an outlet reaction temperature of 240°C (464°F) to 400°C (752°F), preferably 250°C (482°F) to 280°C (536°F), a LHSV of 1 to 50 hr⁻¹, preferably, 15 to 25 hr^{-1,} and a reactor pressure at the last reactor outlet of 1.4 MPa (200 psig) to 5.6 MPa (800 psig), preferably 2.1 MPa (300 psig) to 3.5 MPa (600 psig). The saturation reactors 70 and 72 may be operated in a downflow mode although other reactor configurations and flow regimes may be suitable.

A second saturated effluent stream exits the second saturation reactor 72 in the second saturated effluent line 80 with a greater concentration of naphthenes and a lower concentration of aromatics than in the second combined charge stream in line 78. The second saturated effluent stream in line 80 may be cooled in a second steam generator 82, then by heat exchange with the recycle stream in the recycle line 66 in a recycle oil reactor effluent exchanger 84, then in an optional low-pressure steam generator 86 and then in an optional second reactor effluent cooler 88. The second saturated effluent stream is cooled still further by heat exchange with the separator liquid stream in line 90 in a separator liquid reactor effluent exchanger 92 and then condensed in a product condenser 94 before it is fed to a separator 96. It is envisioned that a third or additional saturation reactors can be employed. Additional reactors may be employed to allow the first saturation reactor 70 or the second saturation reactor 72 to be taken offline for catalyst regeneration or replacement while a pyrolysis feed stream and hydrogen is run to the third or additional reactor without reducing throughput.

The cooled second saturated effluent stream in line 80 separated into a vapor saturated stream in an overhead line 98 extending from an overhead of the separator 96 and a liquid saturated stream in bottoms line 90 extending from a bottom of the separator 96. The vapor saturated stream in line 98 is rich in hydrogen and may be recycled to line 60 for recycle to the saturation reactors 70 and 72 perhaps with a purge or forwarded to a pressure swing adsorption unit or other unit for hydrogen recovery or to provide make-up gas for any unit. The liquid saturated stream is pumped in the bottoms line 90 and heated in the reactor effluent exchanger 92 by heat exchange with the second saturated effluent stream in line 80 and split into the recycle stream in line 66 and a product fractionator feed stream in line 100. The separator 96 is operated at 38°C (100°F) to 66°C (150°F) and 2.1 MPa (300 psig) to 3.1 MPa (450 psig).

The recycle stream in line 66 is taken from the liquid saturated stream in line 90, may be heated in the recycle oil reactor effluent exchanger 84 and is added to the first pyrolysis stream in line 54 and the first hydrogen stream in line 62 to provide the first combined charge stream in line 68 charged to the first saturation reactor 70. It is also envisioned that the recycle stream in line 66 can be heated by heat exchange with the first saturation effluent in line 40. It is also envisioned that the recycle stream in line 66 may be recycled to the first pyrolysis stream in line 54 and to the second pyrolysis stream in line 56. The recycle-to-feed ratio can be 0 to 4 and suitably 0.5 to 1.5.

The product fractionator feed stream in line 100 taken from the liquid saturated stream in line 90 is fed to the product fractionation column 102. The product fractionation feed stream in line 100 is fractionated in the product fractionation column 102 to remove C6 naphthenes in the bottoms stream and n-hexane in the overhead liquid stream. The product fractionation column 102 is operated to separate two fractions, a product overhead stream rich in cyclopentane and normal hexane and particularly rich in paraffins and a product bottoms stream rich in cyclohexane and particularly rich in naphthenes. The product overhead stream rich in normal hexane is withdrawn from the product fractionation column 102 in an overhead line 104 extending from an overhead of the column. A stripper overhead stream in line 108 may be added to the product overhead stream in line 104 to provide a combined overhead stream in line 110. The combined overhead stream in line 110 may be condensed in a cooler and fed to a product overhead separator 112. A portion of the condensed product overhead stream is recycled to the product fractionation column 102 as reflux through a reflux line and the remaining portion of the condensed product overhead stream is withdrawn through a net product overhead line 114. In one embodiment, the condensed product overhead stream may be forwarded to the steam cracking unit 5. In another embodiment, the condensed product overhead product stream may be fed to the overhead liquid stripper 116 to strip out lights. Hydrogen and C3- hydrocarbons are withdrawn in a net product vapor line 118 from an overhead of the product overhead separator 112 and may be transported to a fuel gas header. If the product fractionation column 102 is operated at lower pressure to enable reboil by low pressure steam, the overhead stream from the product overhead separator 112 may be compressed, cooled and separated in a compressor drum. The compressor drum vapor stream in the overhead line could be transported to the fuel gas header and the compressor drum liquid stream in the bottoms line would be recycled back to the product overhead separator 112.

The product bottoms stream is withdrawn from the product fractionation column 102 in a bottoms line 106 extending from a bottom of the column. A reboil portion of the product bottoms stream flows through a reboiler line 122, a reboiler heater which may include heat exchange with steam and returns heated to the product fractionation column 102. A net product bottoms stream flows through line 124 rich in cyclohexane and other naphthenes which may be cooled in a product bottoms cooler 126 and charged back to the steam cracking unit 5 to produce more light olefins. The product fractionation column 102 operates in bottoms temperature range of 110°C (230°F) to 196°C (385°F) and an overhead pressure of 35 kPa to 700 kPa (gauge).

The condensed product overhead stream in the net product overhead line 114 is stripped in the overhead liquid stripper 116 to assure that light hydrocarbons and hydrogen are removed from the product overhead stream. The condensed product overhead stream is stripped into two streams: a stripper overhead stream rich in C3- hydrocarbons and hydrogen and a stripper bottoms stream rich in C4+ hydrocarbons. The stripper overhead stream is withdrawn in the stripper overhead line 108 extending from an overhead of the overhead liquid stripper and is combined with the product fractionator overhead stream in line 104 to have C3- hydrocarbons removed from both streams together in the combined overhead stream in line 110.

A stripper bottoms stream is withdrawn from overhead liquid stripper column 116 in a bottoms line 128 extending from a bottom of the column. A reboil portion of the stripper bottoms stream flows through a reboiler line 130, a reboiler heater which may include heat exchange with steam and returns heated to the overhead liquid stripper 116. A net stripper bottoms stream flows through line 132 rich in C5 naphthenes and C6 paraffins. The overhead liquid stripper column 116 operates in bottoms temperature range of 55°C (131°F) to 138°C (280°F) and an overhead pressure of 35 kPa to 700 kPa (gauge).

The net stripper bottoms stream may be routed to an iso-normal separation unit 134 that separates iso-paraffins from normal paraffins in line 132 to provide a normal paraffin rich stream in line 138. The normal paraffins stream in line 138 can be routed to the stream cracking unit 5 as excellent steam cracker feed. The iso-paraffins rich stream in line 138 can be further processed such as in an isomerization unit to convert iso-paraffins to normal paraffins to provide a superb steam cracker feed. Alternatively, the net stripper bottoms stream may be routed to the stream cracking unit 5.

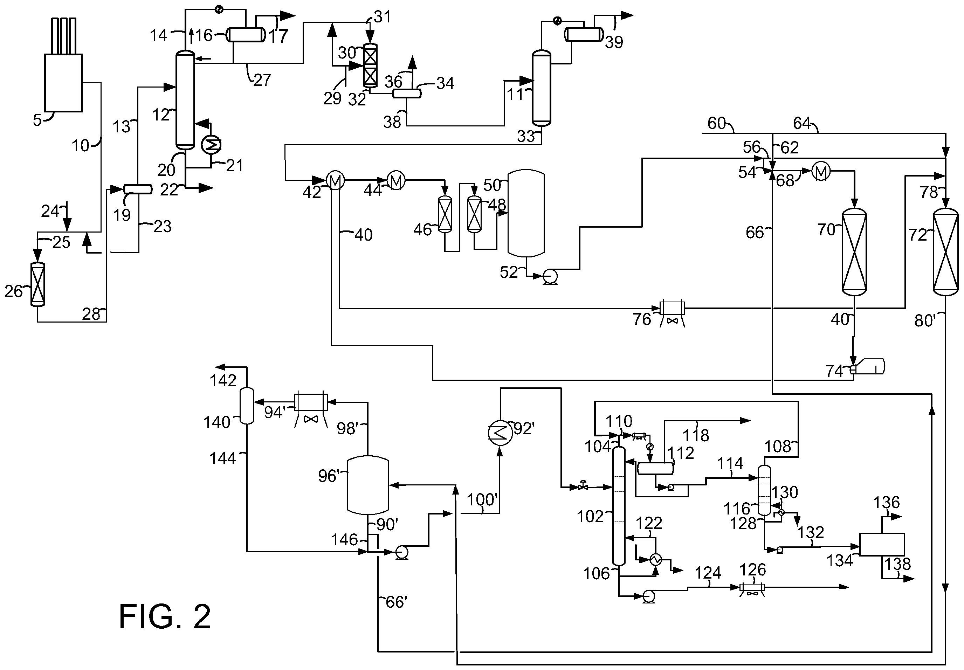

FIG. 2 shows an embodiment of the process in which a hot separator is used to produce the recycle stream 66'. Elements in FIG. 2 with the same configuration as in FIG. 1 will have the same reference numeral as in FIG. 1. Elements in FIG. 2 which have a different configuration as the corresponding element in FIG. 1 will have the same reference numeral but designated with a prime symbol ('). The configuration and operation of the embodiment of FIG. 2 is essentially the same as in FIG. 1.

The second saturated effluent stream in line 80' be taken directly or indirectly to a hot separator 96' with none or some heat exchange in FIG. 1. A hot vapor saturated stream from an overhead line 98' extending from an overhead of the hot separator 96' can be cooled in the product condenser 94' and fed to a cold separator 140. The cold separator separates the hot vapor saturated stream into a cold vapor saturated stream in the overhead line 142 extending from an overhead of the cold separator 140 which can be routed to hydrogen recovery and a cold liquid saturated stream in a cold separator bottoms line 144 extending from a bottom of the cold separator 140. A pump may be used to transport liquid in line 144.

The recycle stream in line 66' can be taken from the hot saturated liquid stream in the hot separator bottoms line 90' with less or no need for reheating to the reaction temperature for the first saturation reactor 40. A remaining net hot saturated liquid stream in a net line 146 taken from the hot saturated liquid stream in the hot separator bottoms line 90' may be combined with the cold saturated liquid stream in line 144 from a bottom of the cold separator 140 to provide a product fractionator feed stream in line 100'. The product fractionator feed stream in line 100' may be cooled in the exchanger 92' and fed to the product fractionation column 102. The rest of FIG. 2 as is described for FIG. 1.

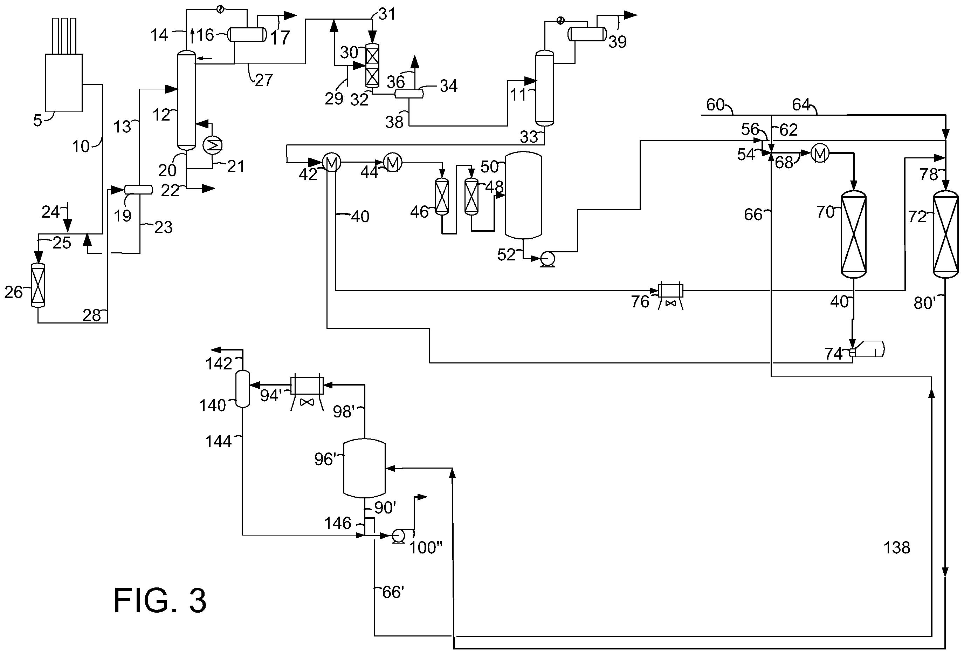

FIG. 3, which is not within the scope of the invention, shows a process in which the combined saturated liquid streams are charged to the steam cracking unit 5 instead of being fractionated. Elements in FIG. 3 with the same configuration as in FIG. 2 will have the same reference numeral as in FIG. 2. Elements in FIG. 3 which have a different configuration as the corresponding element in FIG. 2 will have the same reference numeral but designated with a double prime symbol ("). The configuration and operation of the embodiment of FIG. 3 is essentially the same as in FIG. 2.

The net hot saturated liquid stream in line 146 taken from the hot saturated liquid stream in bottoms line 90' and the cold saturate liquid stream in the cold separator bottoms line 144 combined in line 100" can be taken directly to the stream cracking unit 5 instead of undergoing fractionation. The rest of FIG. 3 as is described for FIG. 2.

The foregoing description provides a process for saturating aromatics in a pyrolysis stream which may make it suitable for steam cracking feed.

### EXAMPLE

A simulation of the disclosed process was run with 5,670 kg/hr (12500 lb/hr) of make-up hydrogen with 43% of the pyrolysis stream split to the first reactor and the balance to the second reactor. Reactor inlet temperatures were 163°C (325°F) and reactor outlet temperatures were 274°C (525°F) and 267°C (512°F), respectively. Reactor pressure was 2.8 MPa (400 psig), the hydrogen-to-hydrocarbon ratio was 0.23 at each reactor outlet, LHSV was 20 hr⁻¹ and the recycle-to-feed ratio was 1.1 on a volume basis. Conversion across both reactors was 97%. The component mass flow rates are provided in Table 2.

**Table 2**

| Components | Mass flow rate (kg/hr (lb/hr)) | | Yield (%) |
|---|---|---|---|
| | Fresh Feed | Net Column Inlet & Separator Overhead | |
| Isopentane | 5670 (13161) | 5670 (13160) | 0 |
| N-Pentane | 7932 (17488) | 7932 (17487) | 0 |
| Cyclopentane | 9290 (20481) | 9290 (20481) | 0 |
| N-Hexane | 2593 (5717) | 2593 (5717) | 0 |
| Cyclohexane | 6725 (14827) | 41915 (92406) | 523 |
| Benzene | 32695 (72081) | 35 (78) | -100 |
| N-Heptane | 436 (961) | 436 (961) | 0 |
| Methylcyclohexane | 1591 (3508) | 22109 (48741) | 1290 |
| Toluene | 19360 (42682) | 107 (236) | -99 |
| 3-Methylheptane | 510 (1124) | 510 (1124) | 0 |
| N-Octane | 695 (1532) | 695 (1532) | 0 |
| 1,1-Dimethylcyclohexane | 1797 (3962) | 7008 (15449) | 290 |
| Ethylcyclohexane | 0.45 (1) | 8561 (18874) | 1887338 |
| Ethylbenzene | 6368 (14040) | 3.2 (7) | -100 |
| Xylenes | 6833 (15065) | 170 (374) | -98 |
| N-Nonane | 519 (1145) | 519 (1145) | 0 |
| TOTAL AROMATICS | 65328 (144024) | 386 (851) | -99 |
| TOTAL NAPHTHENES | 19404 (42778) | 88882 (195951) | 358 |

Table 2 reveals that total aromatics was reduced by 99% while naphthenes increased by over three-fold. Paraffins were insubstantially converted.

In the foregoing, all temperatures are set forth in degrees Celsius and, all parts and percentages are by weight, unless otherwise indicated.

## Claims

1. A process for saturating a pyrolysis gas stream comprising:
splitting the pyrolysis gas into at least a first pyrolysis gas stream and a second pyrolysis gas stream;
adding a first hydrogen stream to the first pyrolysis gas stream;
saturating aromatics in said first pyrolysis gas stream over a saturation catalyst to provide a first saturated effluent stream;
adding a second hydrogen stream to the second pyrolysis gas stream; and
saturating aromatics in said second pyrolysis gas stream over a saturation catalyst to provide a second saturated effluent stream;
wherein the process further comprises:
mixing a recycle stream taken from the second saturated effluent stream with the first pyrolysis gas stream and the first hydrogen stream; wherein the second saturated effluent stream is separated into a vapor saturated stream and a liquid saturated stream and the recycle stream is taken from the liquid saturated stream; and
fractionating a fractionator feed stream taken from the liquid saturated stream to produce a product bottoms stream rich in cyclohexane and a product overhead stream rich in n-hexane.

2. The process of claim 1 further comprising mixing the first saturated effluent stream with the second pyrolysis gas stream and the second hydrogen stream.

3. The process of claim 2 further comprising cooling the first saturated effluent stream before mixing the first saturated effluent stream with the second pyrolysis gas stream and the second hydrogen stream.

4. The process of claim 1 further comprising cooling the vapor saturated stream and separating the vapor saturated stream into a cold vapor saturated stream and a cold liquid saturated stream and combining said cold liquid saturated stream with said fractionator feed stream taken from said liquid saturated stream.

5. The process of claim 1 further comprising charging the product overhead stream to an iso-normal separation unit and/or an isomerization unit.

6. The process of claim 1 further comprising charging the product bottoms stream to a steam cracking unit.

## Patentansprüche

1. Verfahren zum Sättigen eines Pyrolysegasstroms, umfassend:
Aufspalten des Pyrolysegases in mindestens einen ersten Pyrolysegasstrom und einen zweiten Pyrolysegasstrom;
Hinzufügen eines ersten Wasserstoffstroms zum ersten Pyrolysegasstrom;
Sättigen von Aromaten in dem genannten ersten Pyrolysegasstrom über einem Sättigungskatalysator, um einen ersten gesättigten Abflussstrom bereitzustellen;
Hinzufügen eines zweiten Wasserstoffstroms zum zweiten Pyrolysegasstrom; und
Sättigen von Aromaten in dem genannten zweiten Pyrolysegasstrom über einem Sättigungskatalysator, um einen zweiten gesättigten Abflussstrom bereitzustellen;
wobei das Verfahren ferner umfasst:
Mischen eines Rückführungsstroms, der aus dem zweiten gesättigten Abflussstrom entnommen wird, mit dem ersten Pyrolysegasstrom und dem ersten Wasserstoffstrom; wobei der zweite gesättigte Abflussstrom in einen dampfförmigen gesättigten Strom und einen flüssigen gesättigten Strom getrennt wird und der Rückführungsstrom aus dem flüssigen gesättigten Strom entnommen wird; und
Fraktionieren eines Fraktionier-Zufuhrstroms, der aus dem flüssigen gesättigten Strom entnommen wird, um einen Produkt-Bodenstrom, der reich an Cyclohexan ist, und einen Produkt-Überkopfstrom, der reich an n-Hexan ist, zu erzeugen.

2. Verfahren nach Anspruch 1, ferner umfassend das Mischen des ersten gesättigten Abflussstroms mit dem zweiten Pyrolysegasstrom und dem zweiten Wasserstoffstrom.

3. Verfahren nach Anspruch 2, ferner umfassend das Kühlen des ersten gesättigten Abflussstroms vor dem Mischen des ersten gesättigten Abflussstroms mit dem zweiten Pyrolysegasstrom und dem zweiten Wasserstoffstrom.

4. Verfahren nach Anspruch 1, ferner umfassend das Kühlen des dampfförmigen gesättigten Stroms und das Trennen des dampfförmigen gesättigten Stroms in einen kalten dampfförmigen gesättigten Strom und einen kalten flüssigen gesättigten Strom und das Vereinigen des genannten kalten flüssigen gesättigten Stroms mit dem genannten Fraktionier-Zufuhrstrom, der aus dem genannten flüssigen gesättigten Strom entnommen wird.

5. Verfahren nach Anspruch 1, ferner umfassend das Zuführen des Produkt-Überkopfstroms zu einer Iso-Normal-Trenneinheit und/oder einer Isomerisierungseinheit.

6. Verfahren nach Anspruch 1, ferner umfassend das Zuführen des Produkt-Bodenstroms zu einer Dampfcrackeinheit.

## Revendications

1. Procédé de saturation d'un flux de gaz de pyrolyse comprenant :
la division du gaz de pyrolyse en au moins un premier flux de gaz de pyrolyse et un second flux de gaz de pyrolyse ;
l'ajout d'un premier flux d'hydrogène au premier flux de gaz de pyrolyse ;
la saturation des aromatiques dans ledit premier flux de gaz de pyrolyse sur un catalyseur de saturation pour obtenir un premier flux d'effluent saturé ;
l'ajout d'un second flux d'hydrogène au second flux de gaz de pyrolyse ; et
la saturation des aromatiques dans ledit second flux de gaz de pyrolyse sur un catalyseur de saturation pour obtenir un second flux d'effluent saturé ;
dans lequel le procédé comprend en outre :
le mélange d'un flux de recyclage provenant du second flux d'effluent saturé avec le premier flux de gaz de pyrolyse et le premier flux d'hydrogène ; dans lequel le second flux d'effluent saturé est réparti en un flux saturé de vapeur et un flux saturé liquide et le flux de recyclage provient du flux saturé liquide ; et
le fractionnement d'un flux d'alimentation du fractionneur provenant du flux saturé liquide pour produire un flux de fond de produit riche en cyclohexane et un flux de tête de produit riche en n-hexane.

2. Procédé selon la revendication 1 comprenant en outre le mélange du premier flux d'effluent saturé avec le second flux de gaz de pyrolyse et le second flux d'hydrogène.

3. Procédé selon la revendication 2 comprenant en outre le refroidissement du premier flux d'effluent saturé avant le mélange du premier flux d'effluent saturé avec le second flux de gaz de pyrolyse et le second flux d'hydrogène.

4. Procédé selon la revendication 1 comprenant en outre le refroidissement du flux saturé de vapeur et la séparation du flux saturé de vapeur en un flux saturé de vapeur froide et un flux saturé liquide froid et la combinaison dudit flux saturé liquide froid avec ledit flux d'alimentation du fractionneur provenant dudit flux saturé liquide.

5. Procédé selon la revendication 1 comprenant en outre le chargement du flux de tête de produit dans une unité de séparation iso-normale et/ou une unité d'isomérisation.

6. Procédé selon la revendication 1, comprenant en outre le chargement du flux de fond de produit dans une unité de vapocraquage.
